Europäisches Patentamt

European Patent Office

Office européen des brevets

(19)

(11) Publication number: **0 226 397 B1**

## (12) EUROPEAN PATENT SPECIFICATION

(45) Date of publication of patent specification: **18.12.91**

(51) Int. Cl.⁵: **A61M 25/01, A61M 25/02**

(21) Application number: **86309448.8**

(22) Date of filing: **04.12.86**

(54) **Disposable catheter insertion apparatus.**

(30) Priority: **06.12.85 JP 274573/85**

(43) Date of publication of application:
**24.06.87 Bulletin 87/26**

(45) Publication of the grant of the patent:
**18.12.91 Bulletin 91/51**

(84) Designated Contracting States:
**AT BE CH DE ES FR GB IT LI LU NL SE**

(56) References cited:
**EP-A- 0 090 687**
**WO-A-83/01575**
**DE-C- 3 100 442**
**FR-A- 2 522 507**
**GB-A- 2 033 236**

(73) Proprietor: **SHERWOOD MEDICAL COMPANY**
**1831 Olive Street**
**St. Louis, MO 63103(US)**

(72) Inventor: **Kurimoto, Munehito**
**No. 2434-6, Asaba, Asabacho**
**Iwata-Gun, Shizuoka Pref.(JP)**

(74) Representative: **Porter, Graham Ronald et al**
**C/O John Wyeth & Brother Limited Huntercombe Lane South Taplow**
**Maidenhead Berkshire, SL6 0PH(GB)**

## Description

The present invention relates to a disposable catheter insertion apparatus for inserting a catheter under sterile conditions into a blood vessel.

Prior art devices for inserting a plastic catheter through a tubular needle into a blood vessel are known; one example is disclosed in Japanese laid open patent publication Sho 56-20023. The catheter is usually contained in a sealed casing or sheath for sterility and the sheath may be provided with an introducer to be attached to the proximal end of a cannula.

The applicants are aware of the following disadvantages with prior art devices.

After insertion of the catheter into the blood vessel it is usually impossible to remove the introducer and cannula from the end of the catheter because of the proximal connector thereon. One solution is to attach these blood stained components to the proximal connector but this is unsightly and dangerous and may moreover cause infection and disease. Furthermore, the blood stained introducer and cannula are a hinderance in securing the catheter to the skin.

A known introducer comprises a stainless steel tube in a plastics moulding. The stainless steel tube is necessary to reduce sliding resistance to a minimum so that the operator can sense actual resistance of the catheter in the blood vessel. If the sliding resistance is great there is a danger that the operator may unexpectedly force the catheter through the wall of the blood vessel; this occurence is not infrequent. There is an additional danger that plastic may be abraded and the resulting fine powder carried into the blood vessel by sticking to the catheter. This composite introducer is also difficult to manufacture.

The introducer is usually non transparent and the catheter sheath is usually only semi-transparent; it is therefore difficult to determine the length of catheter inserted into the blood vessel.

The catheter and an extension tube thereof are integrally constituted. It is often necessary to disconnect and reconnect infusion apparatus during long term placement of the catheter and this can lead to loose fitting and cracking of the proximal catheter connector with consequent loss of sterility and reliability. If the connector is damaged the catheter may have to be replaced.

The length of catheter remaining outside the blood vessel may be considerable and the excess must be gathered into a loop which is generally inconvenient and awkward.

The sheath for the catheter is often a plastic bag through which the catheter is gripped for insertion; the catheter must be held in the inserted condition whilst the bag is pulled back for a second insertion stroke and this is generally inconvenient and difficult.

FR-A-2 522 507 discloses a catheter comprising a flexible tube in which may be inserted, at the time of placement into the vein, a rigid mandrel, which may subsequently be withdrawn by sliding it inside the tube, said catheter being characterised in that it comprises, on the proximal side, a stopper or connector fast with the mandrel, that the connector has an internal seat for receiving the end of a syringe and that an internal passage is incorporated in the connector, between the bottom of the seat and the proximal end of the mandrel, the assembly being such that a clear space is left at the proximal end of the mandrel, on the periphery of the latter.

One object of the present invention is to provide a catheter insertion apparatus which overcomes the aforementioned problems and permits easy, sterile insertion of a catheter into a blood vessel to any desired depth.

According to the invention there is provided a catheter introducer assembly comprising a catheter, a sheath therefor and a cannula, whereby the tubular sheath is telescoped over said catheter and includes an introducer comprising a tubular member having a distal arm extending axially thereof, and a tube attached to said arm and extending proximally into said tubular member and being spaced from the inner wall thereof, said introducer being slidably telescoped on said catheter, for separating the sheath from said catheter responsive to movement of the catheter through said introducer and for inserting said catheter to a selected depth from within said sheath into a blood vessel while maintaining sterility of said catheter until said catheter enters the blood vessel, and the cannula has distal and proximal ends wherein said introducer attaches to the proximal end thereof and the distal end of said cannula can be inserted into a blood vessel to allow the catheter to pass from said introducer into the blood vessel said introducer having a side opening through which the sheath can be pulled and removed from the catheter.

Such an introducer permits use of a catheter contained within a sheath whereby the distal end of the catheter is inserted into the tube and the distal end of the sheath is inserted into the tubular member around said tube. The introducer has a side opening and the sheath can be pulled through this opening thus urging the catheter through the introducer into the blood vessel. This arrangement permits a single continuous installation movement with the flexible sheath under tension; control of the catheter and sensing of resistance in the blood vessel is thereby improved.

The tube and tubular member of the introducer are preferably co-axial and the introducer may incorporate a blood check valve in the tube.

The introducer may be moulded in two substantially symmetrical parts which fit together about the catheter. Such an introducer can be removed after use by splitting.

The invention also provides a catheter extension tube having a capilliary needle on one end thereof for insertion in a catheter, attachment wings proximal of the needle and a connector at the other end thereof.

Such an extension tube permits use of a catheter without the usual proximal connector in which case a non-splittable introducer can be used.

The extension tube can be replaced quickly and easily without disturbing the catheter if, for example, the proximal connector of the tube is damaged after repeated connection and disconnection of infusion apparatus.

The sheath of the invention may have a continuous slit along the length thereof or may have a longitudinal line of weakness, for example a line of incompatible material, so long as the line of weakness permits free splitting of the sheath during the catheter insertion process.

Other features of the invention will be apparent from the following description of a preferred embodiment shown by way of example only in the accompanying drawing in which:-

Figure 1 is an exploded view of a two part introducer employed in the present invention;

Figure 2 shows an introducer assembly comprising the introducer of Figure 1, a catheter and a sheath;

Figure 3 shows a syringe assembly and cannulated needle for use with the present invention;

Figure 4 corresponds to Figure 2 and illustrates a stage in the catheter insertion process; and

Figure 5 illustrates a winged connector for use with the present invention.

The introducer assembly described hereinafter is supplied in a sealed and sterilized container (not shown) and is removed therefrom immediately prior to use.

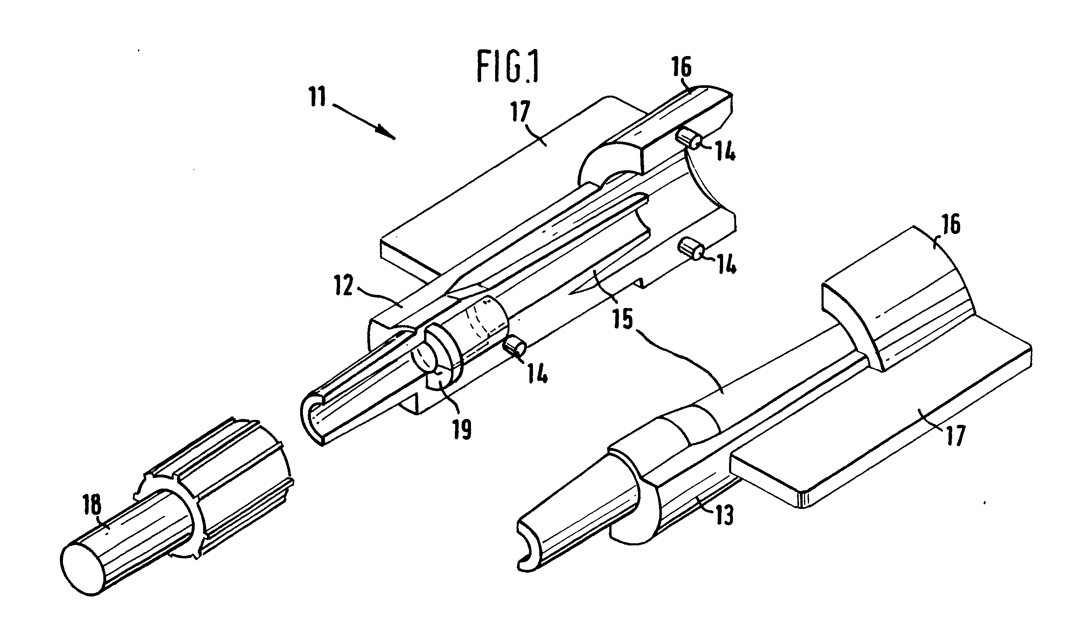

With reference to Figure 1 there is shown an introducer 11 comprising two substantially symmetrical parts 12, 13 and having projections 14 and corresponding holes (not shown) to locate one part relative to the other. The introducer is moulded in a relatively hard plastic and may be transparent.

In the assembled condition the introducer defines a tube 15 having a tubular member or annular shield 16 around the proximal end thereof and finger grips 17. The shield 16 is connected to the mid-portion of the tube 15 by an axially extending arm, of generally "u" shaped section; the underside of the tube is connected to the arm at a part more proximal than the upper surface and the intermediate portion is moulded to provide smooth transition around the tube. The arrangement of tube and arm define an opening in the upper surface of the assembled introducer.

The distal end of the tube has a luer taper, for engagement with a cannula, and is closed by a cap 18. A blood check valve 19 is disposed within the tube and retained in one half thereof as shown; the valve has a slit for removal from the catheter as will be described hereinafter.

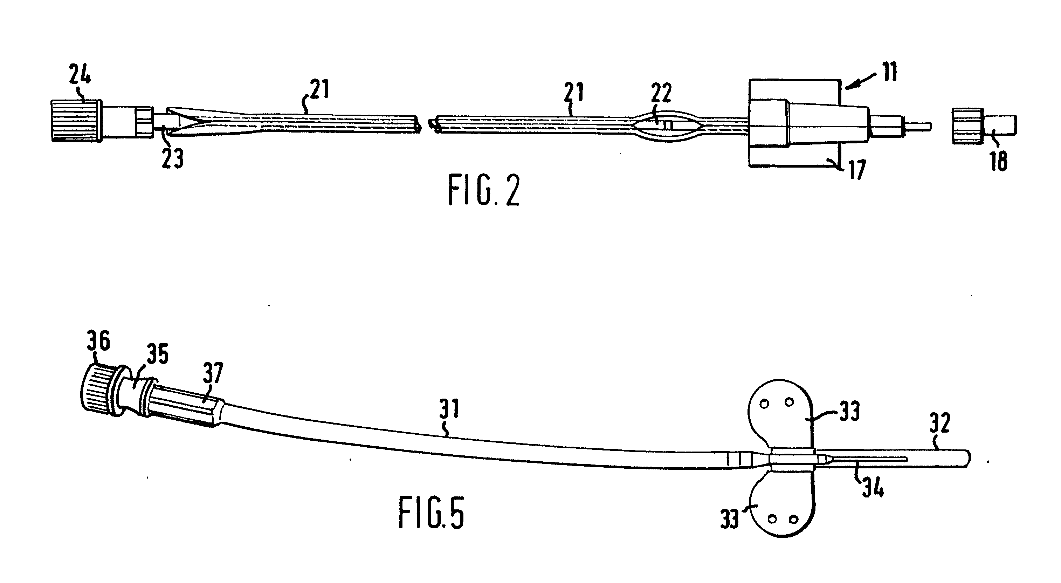

With reference to Figure 2 there is shown an introducer assembly including a sheath 21 of resilient transparent material and having a continuous slit along the length thereof; the slit is closed by the natural resiliency of the material. The material of the sheath is of sufficient resilience to prevent the slit opening when the sheath is bent. The sheath 21 is fitted at one end to the introducer around the proximal end of the tube 15 and within the shield 16.

Within the sheath 21 is a freely slidable catheter 22 having depth graduations thereon; the sheath is shown open for illustration purposes only. The distal end of the catheter is inserted into the tube 15. The proximal end of the catheter has an end fitting 23, which may have a female luer taper, and is closed by a cap 24. The proximal end of the sheath engages the fitting 23 as shown.

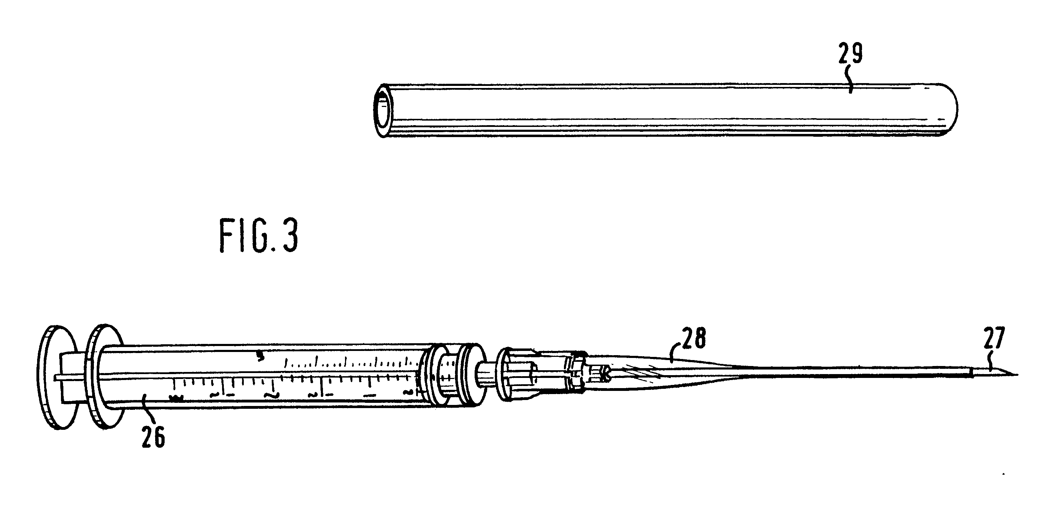

With reference to Figure 3 there is shown a syringe 26 having a needle 27 and surrounding plastics cannula 28. A protective cap 29 is provided for the cannulated needle. The cannula is of translucent or preferably transparent material.

Use of the apparatus will now be described. The syringe and cannulated needle is taken out of the usual sterile pack and the cap 29 removed. The needle is inserted into the desired blood vessel and blood passes into the syringe confirming that the needle is correctly inserted. The cannula 28 is pushed off the tip of the syringe 26 into the blood vessel and the syringe 26 and needle 27 withdrawn for disposal.

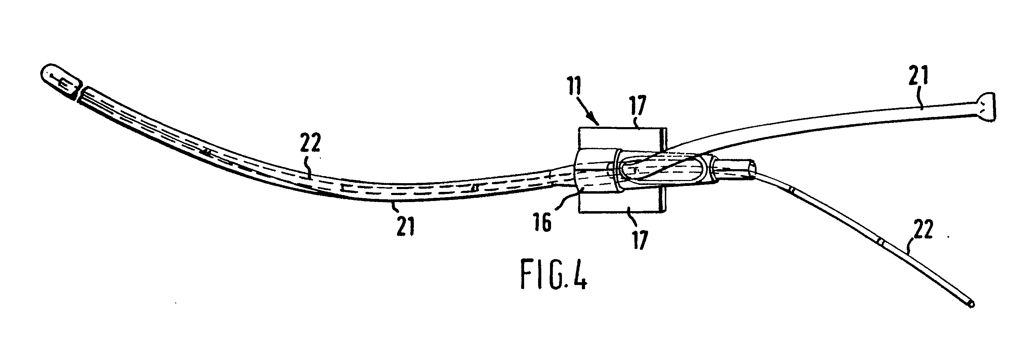

The introducer assembly, which has previously been removed from its sterile pack, is attached to the funnel end of the cannula. The cannula is firmly held by one hand whilst the sheath is moved distally through the shield 16; this initial movement causes the catheter 22 to be advanced through the blood check valve 19 and cannula 28 into the blood vessel. The sheath slit opens around the tube 15 and the distal end of the sheath emerges through the side aperture of the introducer as shown in Figure 4. Further distal movement of the catheter 22 can be accomplished by pulling the free end of the sheath 21; this gives smooth uninterrupted movement of the catheter into the blood vessel; the sheath is maintained under tension giving greater control and feel to the user.

The catheter graduations, best shown in Figure 4, indicate when the desired depth of insertion is reached. The user then removes the remainder of the sheath and separates the introducer and cannula. The introducer is split into the two parts for disposal and the blood check valve removed from the catheter. The cannula is removed from the blood vessel and may be splittable in any known manner for removal from the catheter and disposal; for example the cannula may have a longitudinally extending line of incompatible material. The proximal end of the catheter is attached to other medical apparatus, for example an intravenous drip, in the usual way.

Figure 4 illustrates a catheter without a proximal connector, the sheath 21 is closed at the proximal end thereof to provide a sterile environment for the catheter. After the catheter 22 has been inserted to the desired depth, the excess distal portion may be cut off and the winged extension tube 31 of Figure 5 inserted after the protective cover 32 is first removed. The extension tube 31 has at one end a capilliary extension 34 for connection to the catheter, and at the other end a female connector 35 closed by a removable plug 36 which may be removed for connection of the tube 31 to external apparatus. The wings 33 may be attached to the patient's skin by tape or by sutures through the holes shown. The extension tube 31 has a sleeve 37 at the proximal end thereof to permit the tube to be gripped by forceps without damage thereto.

Where the catheter does not have a proximal connector or where the proximal connector is to be cut off, a one-piece introducer moulding is permissible.

The extension tube 31 can be firmly fixed to the patient's skin without stressing the catheter and can be removed and replaced easily should the female connector become damaged through repeated connection and disconnection of infusion apparatus. The catheter 22 can remain undisturbed in the patient whilst the extension tube is replaced.

The cannula, introducer and sheath are preferably transparent so that the user can visualise the depth markings on the catheter.

The applicants do not intend the scope of the invention to be limited by the above description of a preferred embodiment but only by the claims appended hereto.

## Claims

1. A catheter introducer assembly comprising a catheter (22), a sheath (21) therefor and a cannula (28), wherein the tubular sheath 21 is telescoped over said catheter (22), and includes an introducer (11) comprising a tubular member (16) having a distal arm extending axially thereof, and a tube (15) attached to said arm and extending proximally into said tubular member (16) and being spaced from the inner wall thereof, said introducer (11) being slidably telescoped on said catheter (22), for separating the sheath (21) from said catheter (22) responsive to movement of the catheter (22) through said introducer (11) and for inserting said catheter (22) to a selected depth from within said sheath into a blood vessel while maintaining sterility of said catheter (22) until said catheter enters the blood vessel, and the cannula (28) has distal and proximal ends, wherein said introducer (11) attaches to the proximal end thereof and the distal end of said cannula (28) can be inserted into a blood vessel to allow the catheter (22) to pass from said introducer (11) into the blood vessel, said introducer (11) having a side opening through which the sheath (21) can be pulled and removed from the catheter (22).

2. An assembly as claimed in Claim 1, characterised in that the introducer (11) is moulded in two substantially symmetrical parts which fit together about the catheter (22) and which are removable by splitting.

3. A assembly as claimed in Claim 1, or Claim 2, characterised in that the introducer (11) is transparent, and said sheath (21) is longitudinally splittable from around said tube (15) as the sheath (21) is moved with the catheter through the proximal end of the introducer (11).

4. A catheter introducer assembly as claimed in any one of the preceding Claims characterised in that the introducer (11) incorporates a blood check valve (19) disposed within the inner tube (15).

## Revendications

1. Ensemble d'introduction de cathéter comprenant un cathéter (22), une gaine (21) pour ce cathéter et une canule (28), dans lequel la gaine tubulaire (21) est glissée sur le cathéter (22) et comprend un dispositif d'introduction (11) comprenant un élément tubulaire (16) comportant un bras distal qui s'étend dans le sens axial de cet élément, et un tube (15) attaché à ce bras, s'étendant dans la direction proximale dans l'élément tubulaire (16) et espacé de la paroi interne de ce dernier, le dispositif d'introduction (11) étant reçu à coulissement sur le cathéter (22), pour séparer la gaine (21) du cathéter (22) en réaction au déplacement du cathéter (22) à travers le dispositif d'introduction (11) et pour introduire le cathéter (22) jusqu'à une profondeur sélectionnée à partir de l'intérieur de la gaine dans un vaisseau sanguin tout en maintenant la stérilité de ce cathéter (22) jusqu'à ce qu'il pénètre dans le vaisseau sanguin, et la canule (28) comporte des extrémités distale et proximale, le dispositif d'introduction (11) étant fixé à son extrémité proximale et l'extrémité distale de la canule (28) pouvant être introduite dans le vaisseau sanguin pour permettre au cathéter (22) de passer du dispositif d'introduction (11) dans le vaisseau sanguin, le dispositif d'introduction (11) présentant une ouverture latérale par laquelle la gaine (21) peut être tirée et enlevée du cathéter (22).

2. Ensemble suivant la revendication 1, caractérisé en ce que le dispositif d'introduction (11) est moulé en deux parties en substance symétriques qui s'ajustent ensemble autour du cathéter (22) et qui peuvent être enlevées par séparation des deux parties.

3. Ensemble suivant la revendication 1 ou 2, caractérisé en ce que le dispositif d'introduction (11) est transparent et la gaine (21) peut être divisée longitudinalement depuis sa position située autour du tube (15) lorsqu'elle est déplacée avec le cathéter à travers l'extrémité proximale du dispositif d'introduction (11).

4. Ensemble d'introduction de cathéter suivant l'une quelconque des revendications précédentes, caractérisé en ce que le dispositif d'introduction (11) comprend un clapet de retenue (19) pour le sang disposé dans le tube intérieur (15).

## Patentansprüche

1. Vorrichtung zum Einführen eines Katheters, umfassend einen Katheter (22), ein Futteral (21) für den Katheter und eine Kanüle (28), wobei

das röhrenförmige Futteral (21) über den Katheter (22) gezogen ist und ein Einführelement (11) einschließt, welches ein rohrförmiges Teil (16) mit einem distalen, axial gerichteten Arm und ein mit dem Arm verbundenes Rohr (15), das sich proximal in das rohrförmige Teil (16) erstreckt und von dessen Innenwand beabstandet ist, umfaßt,

das Einführelement (11) verschiebbar auf den Katheter (22) gesteckt ist, und zwar zum Trennen des Futterals (21) von dem Katheter (22) mittels einer Verschiebung des Katheters (22) durch das Einführelement (11) hindurch, und zum Einführen des Katheters (22) aus dem Futteral in ein Blutgefäß bis zu einer bestimmten Tiefe, unter Wahrung der Sterilität des Katheters (22) bis zum Eintritt in das Blutgefäß,

die Kanüle (28) ein distales und ein proximales Ende aufweist, wobei das Einführelement (11) mit deren proximalem Ende verbunden ist und das distale Ende der Kanüle (28) in ein Blutgefäß eingeführt werden kann, um dem Katheter (22) den Übergang von dem Einführelement (11) in das Blutgefäß zu ermöglichen, und

das Einführelement (11) eine seitliche Öffnung aufweist, durch die das Futteral (21) gezogen und vom Katheter (22) entfernt werden kann.

2. Vorrichtung nach Anspruch 1, dadurch gekennzeichnet, daß das Einführelement (11) aus zwei im wesentlichen symmetrischen Teilen geformt ist, die, um den Katheter (22) herumgelegt, zusammenpassen und die durch Trennen entfernbar sind.

3. Vorrichtung nach Anspruch 1 oder 2, dadurch gekennzeichnet, daß das Einführelement (11) transparent ist,

und das Futteral (21) der Länge nach von dem Rohr (15) trennbar ist, indem das Futteral (21) mit dem Katheter durch das proximale Ende des Einführelements (11) geschoben wird.

4. Vorrichtung zum Einführen eines Katheters nach einem der vorhergehenden Ansprüche, dadurch gekennzeichnet, daß das Einführelement (11) ein Blut-Rückschlagventil (19) aufweist, das im inneren Rohr (15) angeordnet ist.

FIG.1

EP 0 226 397 B1

FIG.2

FIG.5

EP 0 226 397 B1

FIG.3

FIG.4

EP 0 226 397 B1